# EUROPEAN PATENT SPECIFICATION

(11) **EP 2 643 038 B1**
(45) Date of publication and mention of the grant of the patent: **21.10.2020**
(21) Application number: 11785704.5
(22) Date of filing: 23.11.2011
(51) Int. Cl.: A61M 15/00, B05B 17/00

(54) **AEROSOL GENERATOR**
AEROSOLGENERATOR
GÉNÉRATEUR D'AÉROSOL

(30) Priority: 24.11.2010 EP 10192385
(43) Date of publication of application: 02.10.2013
(73) Proprietor: PARI Pharma GmbH, 82319 Starnberg (DE)
(72) Inventor: SCHULZ, Harald, 78532 Tuttlingen (DE); HAHN, Michael, 81371 Krailling (DE); HOLZMANN, Philipp, 81371 München (DE); MUTSCHMANN, Dominique, 80333 München (DE)
(74) Representative: Hoffmann Eitle
(86) International application number: PCT/EP2011/070799
(87) International publication number: WO 2012/069531

(56) References cited:
- DE-A1-102007 012 804
- US-A- 5 938 117
- US-A1- 2002 134 374
- US-A1- 2003 140 921
- US-A1- 2009 137 950
- US-B2- 6 983 747

## Description

The present invention relates to an aerosol generator and in particular to an aerosol generator for liquid aerosols with a large liquid reservoir volume which contains a similar volume fraction of liquid and gas. More particularly, the present invention relates to an aerosol generator which contains a liquid with a relatively high viscosity in a liquid reservoir accommodating the liquid to be emitted in form of the aerosol.

Aerosol generators are mainly used for industrial, laboratorial, and/or medical application, as well as in the field of consumer products but are not limited thereto. Especially the generation of efficient, reproducible and constant aerosol output for greater liquid volumes is currently insufficient realized. In all applications which desire a constant output or dose over the complete aerosol generation process and reproducible during every application an optimized aerosol generator are needed.

It has been known that an initial negative pressure (also referred to as starting negative pressure) increases the efficiency and total output rate (TOR) of such aerosol generators (preferable a vibrating mesh or vibrating membrane nebulizer) as taught for example by DE 10 2007 012 804 A1, WO 97/29851 or US 6,983,747 B2 (US 2004/089295 A1 forming the basis for the preamble of claim 1). Further, the use of ampoules holding a volume of 0.2 ml to 6 ml or even more are described in US 2002/0134374 A1. Alternative ampoules containing up to 10 ml of medicament are described in US 2009/0137950 A1.

Another prior art is disclosed in US 2003/0140921 A1.

It might turn out in dose finding studies that relatively high amount of compound need to be delivered to a user. Yet, some liquids (e.g. medical substances or compounds) may not be administered at high concentration for different reasons, high concentration can be related to disadvantageous physicochemical properties for the nebulization, a compound might not be solvable in high concentrations or more general the liquid containing the compound might not be able to carry high concentrations of the compound (i.e. solution, suspension or liposomal drug formulation for inhalation therapy). Thus, in the administration a relatively large volume of liquid needs to be emitted in form of the aerosol. The liquid may contain substance or compounds for example medical liquids, active substances, drugs or further compounds, such as for therapeutic, analytic and/or diagnostic applications. In standard aerosol generators, such as those mentioned in the above documents and although the efficiency and output rate is already increased in those aerosol generators because of the initial negative pressure, a relatively large period of time is required for emitting the entire liquid containing the compound in the form of an aerosol.

Such a long period of time, however, is perceived negative and uncomfortable by a user which can lead to a lower acceptance of the application (e.g. medical aerosol therapy, compromised patient compliance, potentially reduced efficacy of the medical aerosol therapy).

Accordingly, the present invention aims to improve the known aerosol generators in this regard and to provide an aerosol generator that enables the emission of even large amounts of liquid in the form of an aerosol in a shorter period of time.

This objective is resolved by an aerosol generator having the features as defined in claim 1. Embodiments of the present invention are defined in the dependent claims.

The present invention is based on the finding of the present inventors that emitting the liquid in the form of an aerosol while the negative pressure is maintained in a relatively narrow predetermined range, i.e. consistent value the total output rate may be increased significantly resulting in a much shorter period of time required for emitting the liquid. The present invention suggests maintaining the negative pressure in a most efficient and simple way by providing a gas (preferably air) cushion within the liquid reservoir (above the liquid) that acts as a buffer or damper and is sufficiently large to maintain the negative pressure in the intended and optimized range. Hence, no substantial or complicated modifications of the existing aerosol generator are required. Hence, the solution of the present invention leads to an enormous advantage and is easily implementable. The inventors have found that the counterforce to the emission of the liquid through the openings of the membrane needs to be within an optimal range to maintain a high efficiency output rate. A minimum negative pressure is required to prevent the liquid from penetrating through the openings (holes or ducts) which negatively impacts the aerosol generation process. A high counterforce prevents the liquid from passing through the openings. Hence, if the negative pressure increases, which occur during drainage of the medication reservoir from an air tight reservoir, are reduced or even prevented, such increase in the counterforce may be prevented. Thus, the output efficiency may be increased or maintained constant leading to a shortened period of time for the nebulization of the liquid (aerosol generation).

Further, it has been shown that the optimum range for the negative pressure depends on the physical and chemical characteristics of the liquid to be nebulized, such as for example the viscosity (Newtonian fluid or non-Newtonian fluid like thixotrope), surface tension, density, kind of fluid (solution or suspension), solubility, or size of particles in the liquid (suspension). Further the optimum range for the negative pressure may be influenced by the surrounding influencing variables, like ambient air or transportation gas parameters as for example humidity, temperature, pressure.

Furthermore, the efficiency depends on the design of the aerosol generation element and the fluid mechanics device setup. The aerosol generation element (vibrating mesh or membrane) efficiency is dependent on mesh or membrane geometry, number of openings, opening arrangements, oscillation, excitation frequency, bending mode vibration, maximum deflexion, power supply and control unit. The fluid mechanic device setup influences the aerosol deposition in the device by impaction (flow pattern, turbulences) and sedimentation. The third theoretical factor the Brownian molecular movement is in this particle range normally less important. Therefore the flow pattern of the aerosol/gas flow throw the device is highly relevant. In particular for optimized fluid dynamic design of the device a large aerosol mixing chamber with special inlet and outlet valves with an optimized mouthpiece are preferable. (Reference is made to EP 1 227 856 B1)

Accordingly, the present invention suggests an aerosol generator, in particular for aerosols, having a liquid reservoir defining in a sealed and/or closed state a volume V_{R} configured to hold an initial volume (starting volume) of liquid V_{L}. The sealed state is that state in which the reservoir defines a volume V_{R} immediately before use of the aerosol generator, i.e. oscillation of the membrane.. The sealed state is a state in which after sealing of the reservoir the initial volume V_{RI} of the reservoir (e.g. this may be with a lid being placed upon the opening of the reservoir but not being threaded onto the reservoir so as to create the negative pressure (see later)) is increased. Such state is in the following referred to as V_{RN}. The initial volume of liquid V_{L} is the volume that corresponds to a predetermined dose of a compound to be administered in one or more applications (single or multi therapy sessions) of aerosol generation. This initial volume of the compound may be packaged for example in a plurality of single containers or ampoules (blister, vial, vessel, tank) each containing only one dose (the initial volume of liquid). Alternatively, the single containers or ampoules may each contain a certain amount of the drug substance (e.g. liquid, powder or lyophilisate) and this drug is to be mixed with a predetermined volume of a solvent, wherein the predetermined volume of the solvent and the drug contained in the single container upon mixing together form the initial volume of the liquid. In a further alternative, the compound may be provided in a package together with a measuring device to be used for filling the desired dose (initial volume of liquid V_{L}) into the liquid reservoir. This measuring device may be used to measure the drug itself and/or a solvent in order to enable a user to fill the initial volume of liquid into the liquid reservoir. For this purpose, the liquid reservoir may be a reservoir that may be opened and sealed in order to be filled with the liquid. Alternatively, the liquid reservoir may as well be formed by an ampoule that already contains the initial volume of liquid and which is to be inserted into the aerosol generator. Such an ampoule is e.g. described in WO 2007/020073 A1. The ampoule will also be used for

Examples of compounds and active substances that can be used together with the present invention are contained in the non-exhaustive list below.

In one embodiment, the aerosol is a medical and/or pharmaceutical aerosol for the delivery of an active compound. An active compound is a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active pharmaceutical ingredient, drug substance, drug, and the like.

The active compound comprised in the aerosol used for the not claimed method of the invention may be a drug substance which is useful for the prevention, management, diagnoses, or treatment of any disease, symptom, or condition affecting the upper or lower respiratory system (tract), including e.g. mouth, nose, the sinuses and/or the osteomeatal complex, ear, eustachian tube, throat, trachea, airways, lungs , main bronchi's, intermediate bronchus, and alveoli's. The not claimed method of the invention achieves a highly efficient deposition of the active compound in the wished area of the upper or lower respiratory system. Thus, it may be advantageously used for the prevention, management, diagnoses, or treatment of the above diseases, symptoms or conditions. In addition, the present not claimed method may also be used to deliver active compounds to the systemic circulation or to the brain for prevention, management, or treatment of any systemic or brain disease, symptom, or condition.

The aerosol generator of the present invention further comprises a membrane having a plurality of openings and communicating with the liquid reservoir so that the liquid is fed from the liquid reservoir at one side of the membrane. The membrane is configured to be oscillatable which may as one example be achieved by a piezoelectric actuator. When oscillating the membrane, the liquid is transported through the openings, whereby the liquid is emitted in the form of an aerosol on the other side of the membrane. Regarding the configuration of such membranes, the skilled person is referred to EP 0 615 470 B1.

Moreover, the aerosol generator has a negative pressure generating device cooperating with the liquid reservoir so to increase the volume V_{R} of the liquid reservoir in the sealed state of the liquid reservoir to V_{RN} before the membrane is oscillated (that is before starting administration or use). Such a negative pressure generating device may be formed as disclosed in US 6,983,747 B2. Alternatively, the negative pressure generating device may as well be configured as disclosed in WO 2007/020073 A1.

The present inventors have found that the higher the ratio of the initial volume of liquid to the increased volume V_{RN} of the liquid reservoir, the higher the increase of the negative pressure during emission of the liquid in form of the aerosol. This may at a certain level of the negative pressure rapidly decrease the total output rate and, therefore, increase the aerosol generation time. In order to prevent such a rapid increase of the negative pressure within the reservoir during aerosol generation in a most effective and simple manner, the inventors found that an increased volume V_{RN} of the liquid reservoir configured so as to contain a gas cushion of more than 8 ml of gas at an initial volume of liquid V_{L} of at least 4 ml provides for decreased aerosol generation time upon complete emission of the liquid in the liquid reservoir. "Complete emission" in this context means, that once the aerosol generator is started with an initial volume of liquid, the liquid is emitted in one application (therapy session) upon completion of the aerosol generation process. This does not exclude, that a certain amount of liquid remains within the liquid reservoir. In particular, in some cases and depending on the orientation of the membrane, it is conceivable that when a certain minimum volume of liquid within the liquid reservoir is reached, the aerosol generator stops operation because the membrane is not entirely covered by the liquid in the liquid reservoir. Such case will as well be considered as "complete emission", although some liquid remains in the reservoir. In addition, it is to be understood that the volume of gas has to be considered at an initial state because being compared to the initial volume of the liquid. That is, the volume of gas within the liquid reservoir has to be considered before the membrane is oscillated and the compound or liquid within the liquid reservoir is administered, that is before use of the aerosol generator. The gas (air) cushion of the present invention is even advantageous compared to a negative pressure valve or a pressure limiting membrane as disclosed in WO 2007/020073 A1. The latter may encompass problems regarding a reproducible manufacture and, hence, will be more expensive. A negative pressure valve is disadvantageous regarding the hysteresis of the valve and from the view point of hygiene and sterilization. Further, the suggestion of the present invention is advantageous because all mechanical and electro-technical parts of the existing product may be maintained only changing the configuration (volume) of the liquid reservoir to provide for the inventive air/gas cushion.

As previously indicated, the present invention is particularly advantageous, if large amounts of liquid are to be emitted in form of the aerosol and, hence, to liquid reservoirs configured to accommodate an initial volume of liquid V_{L} of at least 6 ml, preferably at least 8 ml with a gas cushion of at least 8 ml.

Further, the inventors have found that the larger the gas cushion, the more constant the negative pressure during the aerosol generation. Accordingly, it is preferred that the volume V_{R} (V_{RN}) of the liquid reservoir is configured to contain more than 11.5 ml, preferably more than 14.5 ml and even more preferred more than 16.5 ml of a gas before the membrane is oscillated, that is before use.

Furthermore, an advantageous relationship between the initial volume of liquid V_{L} and the volume V_{R} (V_{RN}) of the liquid reservoir has been observed. The higher the ratio of the initial liquid within the liquid reservoir to the volume V_{R} of the liquid reservoir, the higher the increase of the negative pressure during aerosol generation. Hence, the effects of the present invention may best be achieved if the ratio of the volume of the liquid reservoir V_{R} to the volume V_{A} of gas in the liquid reservoir is less than 2, preferably less than 1.8 and most preferably 1.6 at an initial volume of the liquid V_{L} of at least 6 ml and most preferred at least 8 ml. This ratio as a matter of course applies after the initial negative pressure has been generated (V_{RN}) and before the membrane is oscillated.

Moreover and according to one embodiment of the present invention, the aerosol generator has a liquid reservoir that contains at least 4 ml, preferably at least 6 ml and more preferably at least 8 ml of liquid. This may for example apply if the liquid reservoir is formed by an ampoule or after a user has poured the liquid into the reservoir just before use.

In addition, it has been found that the efficiency of the aerosol generator decreases at a negative pressure of 35 kPa (350 mbar). Hence, the increased volume of the liquid reservoir V_{RN} is preferably set so that the negative pressure is maintained in a range between 5kPA and 40 kPa (50 mbar and 400mbar), preferably 5kPa and 35kPa (50 mbar and 350 mbar) upon complete emission of the liquid within the liquid reservoir by the membrane. Further, it has been found that in case the liquid within the liquid reservoir is connected to the one side of the membrane without a negative pressure or even with a slight overpressure the liquid enters the openings of the membrane before oscillation. As a result, droplets may form at the other side of the membrane. These droplets adhering to the membrane may have a negative influence on the transient oscillation of the membrane. As a result, the aerosol generation is delayed or may even not be started. For this reason, the lower border has been selected at a negative pressure of 10 kPa (100 mbar) in particular for higher viscous liquids.

Further and in view of the above, it is preferred that the initial negative pressure before the membrane is oscillated resides between 5kPa and 35 kPa (50 and 350 mbar), preferably 10 kPa and 200 kPa (100 mbar and 200 mbar) and even most preferred between 10 kPa and 15 kPa (100 mbar and 150 mbar).

In addition, the optimum negative pressure inter alia depends on the physical and chemical characteristics of the liquid to be emitted in the form of an aerosol. It has been found that particularly with liquids having a higher viscosity of at least 1.5 mPa x s such as for example glycerol 17% in a saline solution (i.e. 1.5% NaCl solution), the efficiency within a negative pressure range of 10 kPa and 35 kPa (100 mbar and 350 mbar) is nearly constant, whereas the efficiency is clearly decreased below and above these values. Accordingly, the present invention is preferably implemented with such highly viscous liquids.

Furthermore, and according to one embodiment, the aerosol generator, in particular the compartment which contains the liquid, comprises a calibration mark indicating the initial volume V_{L} of the liquid to be filled into the liquid reservoir.

As previously indicated and in accordance with one embodiment, the liquid reservoir may have an opening and the aerosol generator further comprises sealing element arranged on that opening to seal the liquid reservoir interfacing the aerosol generator, wherein the negative pressure generating means comprises a slidable element connected to the sealing element in such a way that a movement of the slidable element effects movement of at least one section of the sealing element, whereby the initial volume V_{RI} of the liquid reservoir is increased to V_{RN} to generate the initial negative pressure. Regarding details of this embodiment, the skilled person is referred to US 6,983,747 B2.

Preferably, a rotary element connected to the slidable element is provided such that rotation of the rotary element effects a substantially linear movement of the slidable element.

Alternatively, the liquid reservoir is formed by an ampoule as previously mentioned which is to be inserted into a housing of the aerosol generator and to be pierced open for connection to the one side of the membrane. In this context, the skilled person is referred to WO 2007/020073 A1.

Further embodiments features and advantages of the present invention which may each or together be implemented together with one or more of the above features will become apparent from the following description of a preferred embodiment. This description makes reference to the accompanying drawings, in which
Figure 1 shows an aerosol generator in which the present invention may be implemented;
Figure 2 shows the aerosol generator shown in Figure 1 in an enlarged representation;
Figure 3 shows a graph explaining the relationship between the time period of aerosol generation upon complete emission of the liquid within the liquid reservoir and the initial gas cushion within the liquid reservoir;
Figure 4 shows a graph explaining the relationship between the negative pressure and the time of aerosol generation until complete emission of the liquid from the liquid reservoir;
Figure 5 is a graph showing a relationship between the aerosol generation efficiency (proportional to liquid output rate or total output rate) and the negative pressure; and
Figure 6 is a graph showing the relationship between the period of time for aerosol generation upon complete emission of the liquid and the ratio between the increased volume V_{RN} of the liquid reservoir and the initial volume of liquid within the liquid reservoir.

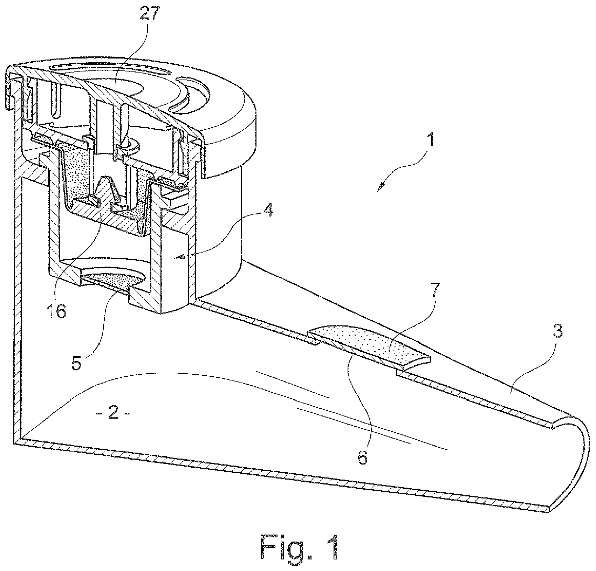

FIG. 1 shows a therapeutic aerosol device 1 with a nebulizing chamber 2, a mouthpiece 3 and a membrane aerosol generator 4 whose oscillating membrane is marked 5 in FIG. 1. The oscillating membrane may, for example, be brought to oscillation by annular piezo elements (not shown), examples of which are described inter alia in WO 97/29851 A1. When in use, the liquid is located on one side of the oscillating membrane 5, see top of FIG. 1, and this liquid is then transported through openings in the oscillating membrane 5 and emitted on the other side of the oscillating membrane 5, see bottom of FIG. 1, as an aerosol into the nebulizing chamber 2. The patient is able to breathe in the aerosol present in the nebulizing chamber 2 at the mouthpiece 3. So that the patient does not have to remove or to put down the therapeutic device from his mouth after inhaling the aerosol, the mouthpiece 3 has an opening 6 sealed by an elastic valve element 7 (exhalation valve). If the patient exhales into the mouthpiece 3 and hence into the nebulizing chamber 2, the elastic valve element 7 opens so that the exhaled air is able to escape from the interior of the therapeutic aerosol. On inhalation, ambient air flows through the nebulizing chamber 2. The nebulizing chamber 2 has an opening sealed (not shown) by a further elastic valve element (inhalation valve). If the patient inhales through the mouthpiece 3 and sucks from the nebulizing chamber 2, the elastic valve element opens so that the ambient air is able to enter into the nebulizing chamber and mixed with the aerosol and leaf the interior of the nebulizing chamber 2 to be inhaled. This will be described in more detail in US 6,962,151.

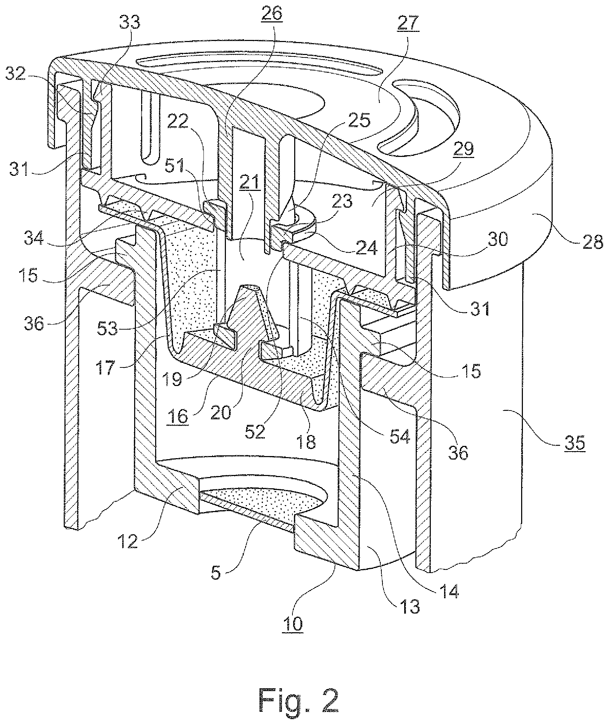

Firstly, however, there follows a description of the structure of the aerosol generator according to the invention with reference to FIG. 2.

The aerosol generator according to FIG. 2 described here as an example comprises a cylindrical storage vessel 10 to supply a liquid that is fed to the membrane 5. As shown in FIG. 2, the oscillating membrane 5 may be arranged in an end wall 12 of the cylindrical liquid reservoir 10 to ensure that the liquid poured into the liquid reservoir comes into direct contact with the membrane 5 when the aerosol generator according to the invention is held in the position shown in FIG. 1. However, other methods may also be used to feed the liquid to the oscillating membrane without any change being necessary to the design of the device according to the invention for the generation of a negative pressure in the liquid reservoir. However, due to the compact design of the aerosol generator according to FIGS. 1 and 2, this embodiment is particularly advantageous.

On the side facing the end wall 12, the cylindrical liquid container 10 is open. The opening is used to pour the liquid into the liquid reservoir 10. Slightly below the opening on the external surface 13 of the peripheral wall 14 there is a projection 15 which serves as a support when the liquid container is inserted in an appropriately embodied opening in a housing 35.

The open end of the liquid container 10 is closed by a flexible sealing element 16. The sealing element 16 lies on the end of the peripheral wall 14 of the liquid container 10 and extends in a pot-shaped way into the interior of the liquid container 10 whereby a conically running wall section 17 is formed in the sealing element 16 and closed off by a flat wall section 18 of the sealing element 16. As will be explained again below, forces act via the flat wall section 18 on the sealing element 16 and so the flat wall section 18 is preferably thicker than the other sections of the sealing element 16. On the perimeter of the flat wall section 18, there is a distance to the conical wall section 17 so that the conical wall section 17 may be folded when the flat wall section 18 is moved upwards, relative to the representation in FIG. 2.

On the side of the flat wall section 18 facing away from the interior of the liquid container, there is a projection comprising a truncated cone section 19 and a cylindrical section 20. This design enables the projection to be introduced and latched into an opening adapted to match the cylindrical section since the flexible material of the sealing element 16 permits the deformation of the truncated cone section 19.

According to the invention, the aerosol generator 4 comprises a slidable sleeve 21 equipped with an opening of this type which is substantially a hollow cylinder open on one side. The opening for the attachment of the sealing element 16 is embodied in an end wall of the slidable sleeve 21. When the truncated cone 19 has latched into place, the end wall of the slidable sleeve 21 containing the opening lies on the flat sealing element wall section 18. The latching of the truncated cone 19 into the slidable sleeve enables forces to be transmitted from the slidable sleeve 21 onto the flat wall section 18 of the sealing element 16 so that the sealing section 18 follows the movements of the slidable sleeve 21 in the direction of the central longitudinal axis of the liquid container 10.

In a generalized form, the slidable sleeve 21 may be seen as a slidable element, which may, for example, also be implemented as a slidable rod which may be stuck-on or inserted in a drill hole. Characteristic of the slidable element 21 is the fact that it may be used to apply a substantially linearly directed force onto the flat wall element 18 of the sealing element 16. Overall, the decisive factor for the mode of operation of the aerosol generator according to the invention is the fact that a slidable element transmits a linear movement onto the sealing element so that an increase in volume occurs within the liquid reservoir 10. Since the liquid reservoir 10 is otherwise gas-tight, this causes a negative pressure to be generated in the liquid reservoir 10.

The sealing element 16 and the slidable element 21 may be produced in one piece, i.e. in one operation, but from different materials. The production technology for this is available so that a one-piece handlable component for the aerosol generator according to the invention is created which may be produced in a fully automatic production step.

The slidable sleeve 21 is open on the end facing the drill hole for the truncated cone but at least two preferably diametrically opposite lugs 22 and 23 protrude radially into the interior of the slidable sleeve 21. A collar 24 encircling the slidable sleeve extends radially outwards. While the collar 24 is used as a support for the slidable sleeve 21 in the position shown in FIG. 2, the projections 22 and 23 protruding into the interior of the slidable sleeve 21 are used to absorb the forces acting on the slidable sleeve 21 in particular parallel to the central longitudinal axis. According to the invention, these forces are generated by means of two spiral grooves 25 which are located on the outside of the peripheral wall of a rotary sleeve 26.

The device according to the invention may also be implemented with one of the projections 22 or 23 and one groove 25. However, preference should be given to a uniformly distributed arrangement of two or more projections and a corresponding number of grooves.

The rotary sleeve 26 is also a cylinder open on one side whereby the open end is arranged in the slidable sleeve 21 and is hence facing the truncated cone 19 enabling the truncated cone 19 to penetrate the rotary sleeve 26. In addition, the rotary sleeve 26 is arranged in the slidable sleeve 21 in such a way that the projections 22 and 23 lie in the spiral grooves 25. The inclination of the spiral groove 25 is designed so that, when the rotary sleeve 26 is rotated in relation to the slidable sleeve 21, the projections 22 and 23 slide along the spiral grooves 25 causing a force directed parallel to the central longitudinal axis to be exerted on the sliding projections 22 and 23 and hence on the slidable sleeve 21. This force displaces the slidable sleeve 21 in the direction of the central longitudinal axis so that the sealing element 16 which is latched into the slidable sleeve's drill hole by means of the truncated cone is also substantially displaced parallel to the central longitudinal axis.

The displacement of the sealing element 16 in the direction of the central longitudinal axis of the liquid container 10 generates a negative pressure in the liquid container 10, determined inter alia by the distance by which the slidable sleeve 21 is displaced in the direction of the central longitudinal axis. The displacement causes the initial volume V_{RI} of the gas-tight liquid container 10 to increase to the volume V_{RN} and thereby a negative pressure to be generated. This displacement is in turn defined by the design of the spiral grooves 25 in the rotary sleeve 26. In this way, the aerosol generator according to the invention ensures that the negative pressure in the liquid reservoir 10 may be generated in the relevant areas by means of simple structural measures. To ensure that the forces to be applied to generate the negative pressure when handling the device remain low, the rotary sleeve 26 is embodied in one piece with a handle 27 whose size is selected to enable the user to rotate the handle 27, and hence the rotary sleeve 26, manually without great effort. The handle 27 substantially has the shape of a flat cylinder or truncated cone which is open on one side so that a peripheral gripping area 28 is formed on the external periphery of the handle 27 which is touched by the user's hand to turn the handle 27. Due to the design of the spiral grooves 25 and the overall comparatively short distance to be travelled by the slidable sleeve 21 in the longitudinal direction to generate a sufficient negative pressure, it is only necessary to turn the handle 27 and hence the rotary sleeve 26 through a comparatively small angle. In preferred embodiments, this angle of rotation lies within a range from 45° to 360°. This embodiment makes a significant contribution to the ease of handling of the device according to the invention and an aerosol generator or therapeutic aerosol equipped therewith.

In order to create a unit which may be operated simply and uniformly from the slidable sleeve 21 and the rotary sleeve 26 including the handle 27, the example of an embodiment of the aerosol generator described here has a bearing sleeve 29 for bearing the slidable sleeve 21, which substantially comprises a flat cylinder open on one side. The diameter of the peripheral wall 30 of the bearing sleeve 29 is smaller than the internal diameter of the handle 27 and, in the example of an embodiment described, is aligned on the internal diameter of a cylindrical latching ring 31 which is provided concentrically to the gripping area 28 of the handle 27 but with a smaller diameter on the side of the handle 27 on which the rotary sleeve 26 is also arranged. Embodied on the side of the cylindrical latching ring 31 facing the rotary sleeve is a peripheral latching edge 32 which may be brought into engagement with latching lugs 33 situated at intervals on the peripheral wall 30 of the bearing sleeve 29. This enables the handle 27 to be located on the bearing sleeve 29 whereby, as shown in FIG. 2, the handle 27 is placed on the open end of the bearing sleeve 29 and the latching edge 32 is interlatched with the latching lugs 33.

To hold the slidable sleeve 21, an opening is provided in the centre of the sealed end of the bearing sleeve 29 in which the slidable sleeve 21 is arranged, as may be identified in FIG. 2. The collar 24 of the slidable sleeve 21 lies in the position shown in FIG. 2 on the surface of the end wall of the bearing sleeve 29 facing the handle. Extending into the bearing opening are two diametrically opposite projections 51 and 52, which protrude into two longitudinal grooves 53 and 54 on the peripheral surface of the slidable sleeve 21. The longitudinal grooves 53 and 54 run parallel to the longitudinal axis of the slidable sleeve 21. The guide projections 51 and 52 and the longitudinal grooves 53 and 54 provide anti-rotation locking for the slidable sleeve 21 so that the rotational movement of the rotary sleeve 26 results not in rotation but in the linear displacement of the slidable sleeve 21. As is evident from FIG. 2, this ensures that the slidable sleeve 21 is held in the combination of the handle 27 and the bearing sleeve 29 in an axially displaceable way but locked against rotation. If the handle 27 is now rotated in relation to the bearing sleeve 29, the rotary sleeve 26 also rotates in relation to the slidable sleeve 21 whereby the sliding projections 22 and 23 move along the spiral grooves 25. This causes the slidable sleeve 21 to be displaced in an axial direction in the opening of the bearing sleeve 29.

It is possible to dispense with the guide projections 51 and 52 in the bearing opening and the longitudinal grooves 53 and 54 in the slidable sleeve 21 if the design of the truncated cone 19 and the cylinder sections 20 of the sealing elements 16 and the large-area support for the slidable sleeve 21 holding the truncated cone on the flat sealing element section 18 achieves anti-rotation locking of the slidable sleeve 21 by means of friction. For this, the sealing element 16 has to be fixed so it is unable to rotate in relation to the bearing sleeve 29.

Provided on the surface of the sealed end of the bearing sleeve 19 facing away from the handle is an annular first sealing lip 34 concentric to the opening holding the slidable sleeve. The diameter of the first sealing lip 34 corresponds to the diameter of the peripheral wall 14 of the liquid container 10. As may be identified from FIG. 2, this ensures that the first sealing lip 34 presses the sealing element 16 on the end of the peripheral wall against the liquid reservoir 10 in such a way that the liquid reservoir 10 is sealed. In addition, the first sealing lip 34 may also fix the sealing element 16 so that it is unable to rotate in relation to the liquid reservoir 10 and the bearing sleeve 29. Due to the materials normally used for the sealing element on the one hand and the other components of the device according to the invention on the other, no excessive force needs to be applied in order to ensure that the aforesaid components of the device according to the invention are unable to rotate in relation to each other.

With the advantageous example of an embodiment described here, the forces required are generated at least to some extent by means of an interaction between the handle 27 and the housing 35 in which the liquid reservoir is embodied as one piece or in which the liquid reservoir 10 is inserted as shown in FIG. 2. In this case, the liquid reservoir 10 inserted in the casing with the peripheral projection 15 lies at intervals on a support 36 in the housing 35 which extends radially into the interior of the housing 35. This enables the liquid reservoir 10 to be easily removed from the housing 35 for purposes of cleaning. Since support is only provided at intervals, openings are provided for ambient air when the patient inhales, as is described in more detail below.

Partially identifiable only in FIG. 2 is the rotary lock, which is implemented by means of the handle 27 on the one hand and the housing 35 on the other. Only shown are the locking projections 62 and 63 on the housing 35. However, there are no special requirements with regard to the design of the rotary lock as far as the device according to invention is concerned for the generation of the negative pressure in the liquid reservoir 10.

According to an embodiment of the present invention, the liquid reservoir 10 is configured to have a volume V_{RN} of at least 12 ml, preferably at least 16 ml and most preferred at least 20 ml so that when for example an amount of 8 ml of liquid to be emitted in the form of an aerosol is contained in (filled or poured into) the liquid reservoir 10, an air cushion of 8 ml is provided. That is, the ratio of the increased volume V_{RN} to the initial volume of liquid V_{L} within the liquid reservoir 10 is at least 2.0 and the ratio between the volume V_{A} of a gas and V_{L} of the liquid is at least 1.0. Yet, it has been shown that a liquid reservoir having an increased volume V_{RN} of around 15.5 ml is more efficient, that a reservoir of around 19.5 ml is even more efficient and that a reservoir of around 22.5 ml even improves over such reservoirs. That is, it is preferred that the ratio between V_{RN} and V_{L} is at least 2.0, more preferred at least 2.4 and most preferred at least 2.8, the ratio between V_{A} and V_{L} preferably being at least 1.0, more preferred at least 1.4 and most preferred at least 1.8. That is the volume of the air cushion is preferably at least 6 ml, more preferred at least 11 ml and most preferred at least 14 ml.

The ratio of the increased volume V_{RN} to the initial volume of liquid V_{L} is at least 2.0. Theoretically an unlimited enlargement of the increased volume V_{RN} of the liquid reservoir 10 will result in a nearly stable negative pressure range. To held an embodiment of the present invention practicable handy the optimum of the ratio of the increased volume V_{RN} to the initial volume of liquid V_{L} is within the range between 2.0 and 4.0 and is preferably between 2.4 and 3.2. Two Examples of the optimum ratio ranges (V_{RN} / V_{L}) for different initial volume of liquid V_{L} between 4 ml and 8 ml will be given in the below tables.

| V_{L} | V_{RN} | ratio (V_{RN} / V_{L} ) |
|---|---|---|
| 4 ml | 8.0 - 16.0 | 2.0 - 4.0 |
| 5 ml | 10.0 - 20.0 | 2.0 - 4.0 |
| 6 ml | 12.0 - 24.0 | 2.0 - 4.0 |
| 8 ml | 16.0 - 32.0 | 2.0 - 4.0 |

| V_{L} | V_{RN} | ratio (V_{RN} / V_{L} ) |
|---|---|---|
| 4 ml | 9.5 - 12.8 | 2.4 - 3.2 |
| 5 ml | 12,0 - 16,0 | 2.4 - 3.2 |
| 6 ml | 14.5 - 19.2 | 2.4 - 3.2 |
| 8 ml | 19.5 - 25.6 | 2.4 - 3.2 |

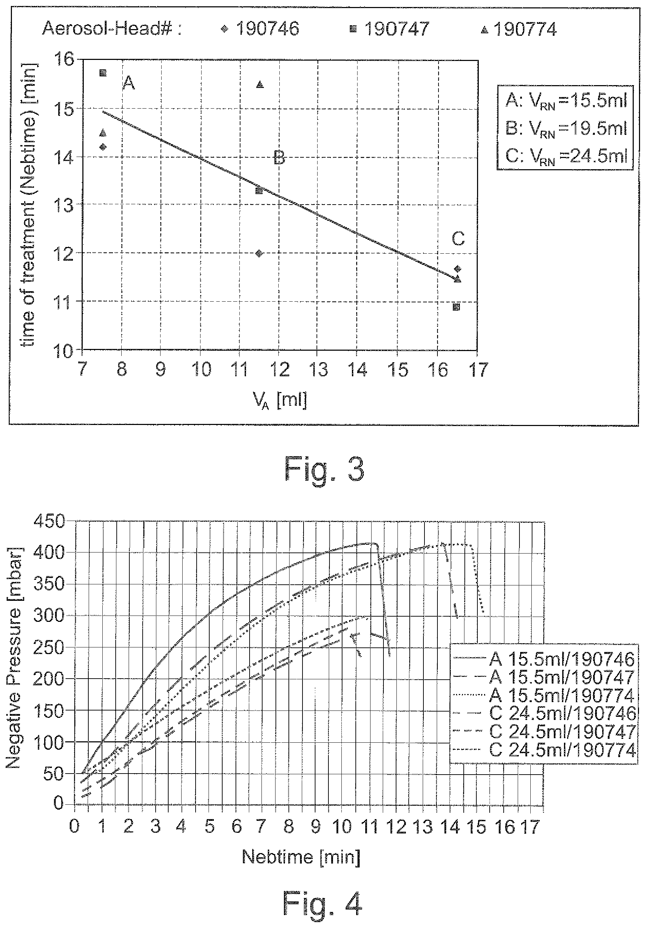

The following Figures 3 to 6 show graphs that represent experimental data proving the effects and advantages of the present invention.

In these examples, the aerosol generator was an investigational eflow (nearly standard) of Pari Pharma GmbH, Germany. The eflow generator has been altered in regard of the volume V_{R} of the liquid reservoir. A first aerosol generator had an initial volume of the liquid reservoir V_{RI} of 13 ml (A), a second one of 17 ml (B), a third one of 22 ml (C) and a fourth one of 20 ml (D). That is the increased volume V_{RN} of the first one had 15.5 ml, the second one 19.5 ml and the third 24.5 ml. It had been measured the time needed from starting the aerosol generator until complete emission, that is termination of the operation of the generator. Further, 8 ml of the liquid were poured into the liquid reservoir 10. As shown in Figure 3 an air cushion of 8 ml results to an aerosol generation time period upon complete emission of 8 ml of the liquid in the liquid reservoir of between 16 and 14 minutes. An air cushion of 12 ml, however, already decreases the aerosol generation time to a range between 13 and approximately 12 minutes. The air cushion of 17 ml further decreases the aerosol generation time to an amount between 12 and 10.

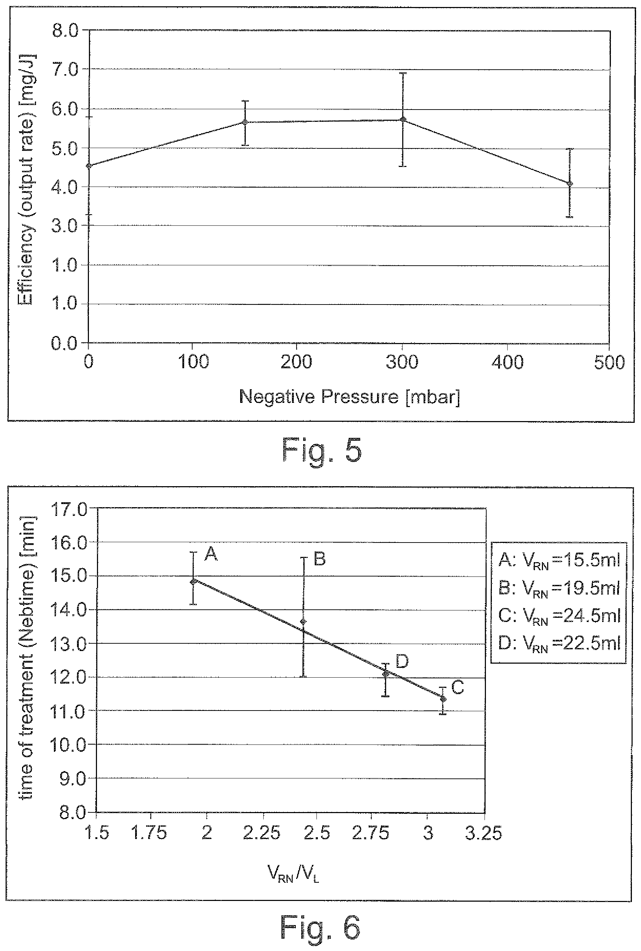

Further, the first (A) and third (C) version of the above aerosol generator had been used together with 8 ml of liquid (i.e. liposomal amikacin). Further, an initial negative pressure of equal to or less than 50 mbar had been generated within the liquid reservoir. In addition, the negative pressure had been measured during the aerosol generation and is shown over the aerosol generation time in Figure 4. That is, Figure 4 shows experimental data comparing the negative pressure range during the aerosol generation time for (C) a liquid reservoir having an increase volume V_{RN} of 24.5 ml and (A) one having an increased volume V_{RH} of 15.5 ml with the initial amount of liquid V_{L} being 8 ml and the initial negative pressure being about 50 mbar. This graph clearly shows that a larger air cushion prevents the negative pressure from increasing above a critical value of 300 mbar. Further, an experiment has been performed with a nearly standard eFlow of PARI Pharma GmbH and the aerosol generator efficiency (proportional to liquid output rate or total output rate) had been measured in dependency of different negative pressures. A liquid (i.e. liposomal amikacin) having a viscosity in the range of 14.5 to 5.5 mPa x s at sheer forces between 1.1 and 7.4 Pa (thixotrope) has been used in the experiment. As shown in Figure 5 the efficiency is optimum in a negative pressure range between 15kPa to 30 kPa (150 mbar to 300 mbar). As may be derived from Figure 5, the efficiency decreases at a negative pressure below approximately 15 kPa (150 mbar) and at a negative pressure of above 30 kPa (300 mbar) Furthermore, the same liquid as in Figure 5 has been used in four different aerosol generators based on the nearly standard eFlow, wherein the first A) one is a modified eFlow with an increased volume V_{RN} of the liquid reservoir of 19.5 ml and filled with 8 ml of the liquid. The liquid was that also used with respect to Figure 5.

The second one had a reservoir with an increased volume V_{RN} of 16 ml filled with 8 ml of the mentioned liquid, the third C) one had an increased volume V_{RN} of 24.5 ml, filled with 8 ml of the mentioned liquid and the fourth one had an increased volume V_{RN} of the liquid reservoir of 22.5 ml filled with 8 ml of the liquid. Figure 6 represents experimental data of these four aerosol generators filled with 8 ml of the substance and shows the aerosol generation time upon complete emission of the liquid within the liquid reservoir in relation to the ratio of the increased volume V_{RN} of the liquid reservoir to the initial volume of liquid V_{L} in the liquid reservoir before use. This graph clearly indicates that with the modified aerosol generator device (A) an aerosol generation time of approximately 16 minutes was required, whereas the aerosol generation time decreases with an increased ratio between the increased volume V_{RN} and the initial volume of liquid and the aerosol generation time could be reduced by approximately 4 minutes with the third device version (C) the aerosol generator to below 12 minutes.

In view of the above, it has been proven that a larger air cushion enables to operate the aerosol generator for a longer time in a most efficient negative pressure range so that the total aerosol generation time may significantly be reduced. Hence, even large amounts of liquid such as 8 ml may be nebulized (emitted in form of aerosol) in a period of time below 12 minutes.

The present invention of an aerosol generator can be used for different liquids, for example for applications in the medical, pharmaceutical, diagnostic and/or analytic fields (e.g. human and veterinary aerosol therapies with drugs, substances or active compounds) as well as for agriculture, humidification, fragrance, hairspray, pyrotechnic, warfare agent, combustion engine, extinguishing, lubrication, adhesive, filtering, cooling, painting, printing, varnishing, coating processes, technologies and systems. Further examples are in the field of cell culture, pollen, herbal, medical, chemical, physical, biological, meteorological, pesticide, fungicide, biocide, toxic, environment, and exposition aerosol applications.

Among the active compounds which may be useful for serving one of the purposes named previously and that may be used together with the present invention, are, for example, substances selected from the group consisting of anti-inflammatory compounds, anti-infective agents, antiseptics, prostaglandins, endothelin receptor agonists, phosphodiesterase inhibitors, beta-2-sympathicomimetica, decongestants, vasoconstrictors, anticholinergics, immunomodulators, mucolytics, anti-allergic drugs, antihistaminica, mast-cell stabilizing agents, tumor growth inhibitory agents, wound healing agents, local anaesthetics, antioxidants, oligonucleotides, peptides, proteins, vaccines, vitamins, plant extracts, phosharimidon, vasoactive intestinal peptide, serotonin receptor antagonists, and heparins, glucocorticoids, anti-allergic drugs, antioxidants, vitamins, leucotriene antagonists, anti-infective agents, antibiotics, antifungals, antivirals, mucolytics, decongestants, antiseptics, cytostatics, immunomodulators, vaccines, wound healing agents, local anaesthetics, oligonucleotides, peptides, proteins and plant extracts. Such compound may be used in the form of a suspension, a solution, in a liposome form, etc.

Examples of potentially useful anti-inflammatory compounds are glucocorticoids and non-steroidal anti-inflammatory agents such as betamethasone, beclomethasone, budesonide, ciclesonide, dexamethasone, desoxymethasone, fluoconolone acetonide, flucinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, dehydroepiandrosterone-sulfate (DHEAS), elastane, prostaglandin, leukotriene, bradykinin antagonists, non-steroidal anti-inflammatory drugs (NSAIDs), such as ibuprofen including any pharmaceutically acceptable salts, esters, isomers, stereoisomers, diastereomers, epimers, solvates or other hydrates, prodrugs, derivatives, or any other chemical or physical forms of active compounds comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam);
- cephalosporins, including cefazolins (cefazolin, cefazedone); cefuroximes (cerufoxim, cefamdole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;

- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including flucytosin, griseofluvin, tonoftal, naftifin, terbinafin, amorolfin, ciclopiroxolamin, echinocandins, such as micafungin, caspofungin, anidulafungin;
- nitrofurans, including nitrofurantoin and nitrofuranzone;
- polyenes, including amphotericin B, natamycin, nystatin, flucocytosine;
- other antibiotics, including tithromycin, lincomycin, clindamycin, oxazolindiones (linzezolids), ranbezolid, streptogramine A+B, pristinamycin aA+B, Virginiamycin A+B, dalfopristin /qiunupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, and pentamidine;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors, Si RNA-based drugs;
- antiseptics, including acridine derivatives, iodine-povidone, benzoates, rivanol, chlorhexidine, quarternary ammonium compounds, cetrimides, biphenylol, clorofene, and octenidine;
- plant extracts or ingredients, such as plant extracts from chamomile, hamamelis, echinacea, calendula, thymian, papain, pelargonium, pine trees, essential oils, myrtol, pinen, limonen, cineole, thymol, mentol, camphor, tannin, alpha-hederin, bisabolol, lycopodin, vitapherole;
- wound healing compounds including dexpantenol, allantoin, vitamins, hyaluronic acid, alpha-antitrypsin, anorganic and organic zinc salts/compounds, salts of bismuth and selen
- interferones (alpha, beta, gamma), tumor necrosis factors, cytokines, interleukines;
- immunmodulators including methotrexat, azathioprine, cyclosporine, tacrolimus, sirolimus, rapamycin, mofetil; mofetil-mycophenolate.
- cytostatics and metastasis inhibitors;
- alkylants, such as nimustine, melphanlane, carmustine, lomustine, cyclophosphosphamide, ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of transition group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, iressa, zactima, poly-ADP-ribose-polymerase (PRAP) enzyme inhibitors, banoxantrone, gemcitabine, pemetrexed, bevacizumab, ranibizumab.

Examples of potentially useful mucolytics are DNase, P2Y2-agonists (denufosol), drugs affecting chloride and sodium permeation, such as N-(3,5-Diamino-6-chloropyrazine-2-carbony)-N'-{4-[4-(2,3-dihydroxypropoxy)-phenyl]butyl}guanidine methanesulfonate (PARION 552-02), heparinoids, guaifenesin, acetylcysteine, carbocysteine, ambroxol, bromhexine, tyloxapol, lecithins, myrtol, and recombinant surfactant proteins.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine. Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, nedocromil, cetrizin, loratidin, montelukast, roflumilast, ziluton, omalizumab, heparinoids and other antihistamins, including azelastine, cetirizin, desloratadin, ebastin, fexofenadin, levocetirizin, loratadin. This list, however, is not exhaustive.

## Claims

1. Aerosol generator having:
a liquid reservoir (10) defining in a sealed state a volume V_{R} holding an initial volume of liquid V_{L};
a membrane (5) having openings, the liquid reservoir (10) being connected to the membrane (5) so as to feed liquid to one side of the membrane, the membrane being oscillatable to transport the liquid through the openings whereby the liquid is emitted in the form of an aerosol on the other side of the membrane, and
a negative pressure generating device (20) cooperating with the liquid reservoir (10) so as to increase an initial volume V_{RI} of the liquid reservoir before the membrane (5) is oscillated to the volume V_{R} of the liquid reservoir in the sealed state, whereby an initial negative pressure is generated in the liquid reservoir,
wherein the volume V_{R} of the liquid reservoir (10) before the membrane (5) is oscillated contains more than 5 ml of gas at an initial volume of the liquid V_{L} of at least 5 ml and
wherein the ratio of the volume of the liquid reservoir (10) V_{R} to the initial volume V_{L} of liquid in the liquid reservoir (10) is more than 2.

2. Aerosol generator according to claim 1, wherein the initial volume of the liquid V_{L} is at least 6 ml, preferably at least 8 ml.

3. Aerosol generator according to any one of the preceding claims, wherein the volume V_{R} of the liquid reservoir (10) contains at least 6 ml of gas, preferably at least 8 ml of gas.

4. Aerosol generator according to any one of the preceding claims, wherein the volume of the liquid reservoir (10) V_{R} is set so that the negative pressure is maintained in a range between 5kPa and 40 kPa, preferably 10 kPa and 35 kPa, most preferred 10 kPa and 30 kPa (50 mbar and 400 mbar, preferably 100 mbar and 350 mbar and most preferred between 100 mbar and 300 mbar) upon complete emission of the liquid within the liquid reservoir (10) by the membrane (5).

5. Aerosol generator according to any one of the preceding claims, wherein the initial negative pressure before the membrane (5) is oscillated resides between 5 kPa and 35 kPa, preferably, 10 kPa and 20 kPa and most preferred between 10 kPa and 15 kPa (50 mbar and 350 mbar, preferably 100 mbar and 200 mbar and most preferred between 100 mbar and 150 mbar).

6. Aerosol generator according to any one of the preceding claims, further comprising a sealing element (16) arranged on an opening of the liquid reservoir (10) to seal the liquid reservoir, the negative pressure generating device comprising a slidable element (21) connected to the sealing element (16) in such a way that a movement of the slidable element (21) effects a movement of at least one section of the sealing element (16) whereby the initial volume V_{RI} of the liquid reservoir (10) is increased to V_{R} to generate the initial negative pressure.

7. Aerosol generator according to claim 6, further comprising a rotary element (26) connected to the slidable element (21) such that rotation of the rotary element effects a substantially linear movement of the slidable element.

8. Aerosol generator according to any one of claims 1 to 5, wherein the liquid reservoir is formed by an ampoule to be inserted into a housing of the aerosol generator and to be pierced open for connection to the one side of the membrane.

9. Aerosol generator according to any one of the preceding claims, wherein the volume V_{R} of the liquid reservoir (10) contains more than 11.5 ml of gas, preferably more than 14.5 ml of gas and most preferable more than 16.5 ml of gas.

10. Aerosol generator according to any one of the preceding claims, wherein the ratio of the volume of the liquid reservoir (10) V_{R} to the initial volume V_{L} of liquid in the liquid reservoir (10) is more than 2.4 and most preferably at least 2.8.

11. Aerosol generator according to any one of the preceding claims, wherein the viscosity of the liquid held by the liquid reservoir (10) is at least 1.5 mPa x s.

12. Aerosol generator according to any one of the preceding claims, wherein the liquid reservoir (10) comprises a calibration mark indicating the initial volume V_{L} of the liquid.

## Patentansprüche

1. Aerosolerzeuger, aufweisend:
einen Flüssigkeitsbehälter (10), der in einem abgedichteten Zustand ein Volumen V_{R} definiert, das ein anfängliches Flüssigkeitsvolumen V_{L} hält;
eine Membrane (5), die Öffnungen aufweist, wobei der Flüssigkeitsbehälter (10) mit der Membrane (5) verbunden ist, um Flüssigkeit an eine Seite der Membrane zuzuführen, wobei die Membrane oszilliert werden kann, um die Flüssigkeit durch die Öffnungen zu transportieren, wodurch die Flüssigkeit in der Form eines Aerosols an die andere Seite der Membrane ausgegeben wird, und
eine negative Druckerzeugungsvorrichtung (20), die mit dem Flüssigkeitsbehälter (10) zusammenwirkt, um ein anfängliches Volumen V_{RI} des Flüssigkeitsbehälters zu erhöhen, bevor die Membrane (5) zu dem Volumen V_{R} des Flüssigkeitsbehälters in dem abgedichteten Zustand oszilliert wird, wodurch ein anfänglicher negativer Druck in dem Flüssigkeitsbehälter erzeugt wird,
wobei das Volumen V_{R} des Flüssigkeitsbehälters (10), bevor die Membrane (5) oszilliert wird, mehr als 5 ml Gas bei einem anfänglichen Flüssigkeitsvolumen V_{L} von mindestens 5 ml enthält und
wobei Verhältnis des Volumens des Flüssigkeitsbehälters (10) V_{R} zu dem anfänglichen Flüssigkeitsvolumen V_{L} in dem Flüssigkeitsbehälter (10) mehr als 2 ist.

2. Aerosolerzeuger nach Anspruch 1, wobei das anfängliche Flüssigkeitsvolumen V_{L} mindestens 6 ml ist, bevorzugt mindestens 8 ml.

3. Aerosolerzeuger nach einem der vorstehenden Ansprüche, wobei das Volumen V_{R} des Flüssigkeitsbehälters (10) mindestens 6 ml Gas enthält, bevorzugt mindestens 8 ml Gas.

4. Aerosolgenerator nach einem der vorstehenden Ansprüche, wobei das Volumen des Flüssigkeitsbehälters (10) V_{R} so eingestellt ist, dass der negative Druck bei vollständiger Ausgabe der Flüssigkeit innerhalb des Flüssigkeitsbehälters (10) durch die Membrane (5) in einer Spanne zwischen 5 kPa und 40 kPa, bevorzugt 10 kPa und 35 kPa und bevorzugter zwischen 10 kPa und 30 kPa (50 mbar und 400 mbar, bevorzugt 100 mbar und 350 mbar und bevorzugter 100 mbar und 300 mbar) gehalten wird.

5. Aerosolerzeuger nach einem der vorstehenden Ansprüche, wobei der anfängliche negative Druck, bevor die Membrane (5) oszilliert wird, zwischen 5 kPa und 35 kPa, bevorzugt 10 kPa und 20 kPa und bevorzugter zwischen 10 kPa und 15 kPa (50 mbar und 350 mbar, bevorzugt 100 mbar und 200 mbar und bevorzugter zwischen 100 mbar und 150 mbar) liegt.

6. Aerosolerzeuger nach einem der vorstehenden Ansprüche, weiter umfassend ein Dichtungselement (16), das an einer Öffnung des Flüssigkeitsbehälters (10) eingerichtet ist, um den Flüssigkeitsbehälter abzudichten, wobei die negative Druckerzeugungsvorrichtung ein schiebbares Element (21) umfasst, das mit dem Dichtungselement (16) auf solche Weise verbunden ist, dass eine Bewegung des schiebbaren Elements (21) eine Bewegung von mindestens einem Abschnitt des Dichtungselements (16) bewirkt, wodurch das anfängliche Volumen V_{RI} des Flüssigkeitsbehälters (10) auf V_{R} erhöht wird, um den anfänglichen negativen Druck zu erzeugen.

7. Aerosolerzeuger nach Anspruch 6, weiter umfassend ein Drehelement (26), das mit dem schiebbaren Element (21) so verbunden ist, dass Drehung des Drehelements eine im Wesentlichen lineare Bewegung des schiebbaren Elements bewirkt.

8. Aerosolerzeuger nach einem der Ansprüche 1 bis 5, wobei der Flüssigkeitsbehälter durch eine Ampulle gebildet ist, die in ein Gehäuse des Aerosolerzeugers einzusetzen und zur Verbindung mit der einen Seite der Membrane aufzustechen ist.

9. Aerosolerzeuger nach einem der vorstehenden Ansprüche, wobei das Volumen V_{R} des Flüssigkeitsbehälters (10) mehr als 11,5 ml Gas, bevorzugt mehr als 14,5 ml Gas und bevorzugter mehr als 16,5 ml Gas enthält.

10. Aerosolerzeuger nach einem der vorstehenden Ansprüche, wobei das Verhältnis des Volumens des Flüssigkeitsbehälters (10) V_{R} zu dem anfänglichen Flüssigkeitsvolumen V_{L} in dem Flüssigkeitsbehälter (10) mehr als 2,4 und bevorzugter mindestens 2,8 ist.

11. Aerosolerzeuger nach einem der vorstehenden Ansprüche, wobei die Viskosität der Flüssigkeit, die von dem Flüssigkeitsbehälter (10) gehalten wird, mindestens 1,5 mPa x s ist.

12. Aerosolerzeuger nach einem der vorstehenden Ansprüche, wobei der Flüssigkeitsbehälter (10) eine Kalibrierungsmarkierung umfasst, die das anfängliche Volumen V_{L} der Flüssigkeit angibt.

## Revendications

1. Générateur d'aérosol présentant :
un réservoir de liquide (10) définissant dans un état fermé de façon étanche un volume V_{R} contenant un volume initial de liquide V_{L} ;
une membrane (5) présentant des ouvertures, le réservoir de liquide (10) étant raccordé à la membrane (5) de manière à alimenter du liquide vers un premier côté de la membrane, la membrane pouvant osciller pour transporter le liquide à travers les ouvertures de telle sorte que le liquide est émis sous la forme d'un aérosol de l'autre côté de la membrane, et
un dispositif générateur de pression négative (20) coopérant avec le réservoir de liquide (10) de manière à augmenter un volume initial V_{RI} du réservoir de liquide avant que la membrane (5) oscille jusqu'au volume V_{R} du réservoir de liquide à l'état fermé de manière étanche, de telle sorte qu'une pression négative initiale est générée dans le réservoir de liquide,
dans lequel le volume V_{R} du réservoir de liquide (10) avant que la membrane (5) oscille contient plus de 5 ml de gaz à un volume initial du liquide V_{L} d'au moins 5 ml et
dans lequel le rapport du volume du réservoir de liquide (10) V_{R} sur le volume initial V_{L} de liquide dans le réservoir de liquide (10) est supérieur à 2.

2. Générateur d'aérosol selon la revendication 1, dans lequel le volume initial du liquide V_{L} est d'au moins 6 ml, de préférence d'au moins 8 ml.

3. Générateur d'aérosol selon l'une quelconque des revendications précédentes, dans lequel le volume V_{R} du réservoir de liquide (10) contient au moins 6 ml de gaz, de préférence au moins 8 ml de gaz.

4. Générateur d'aérosol selon l'une quelconque des revendications précédentes, dans lequel le volume du réservoir de liquide (10) V_{R} est réglé de telle sorte que la pression négative soit maintenue dans une plage comprise entre 5 kPa et 40 kPa, de préférence 10 kPa et 35 kPa, et le plus préférentiellement entre 10 kPa et 30 kPa (50 mbar et 400 mbar, de préférence 100 mbar et 350 mbar et le plus préférentiellement entre 100 mbar et 300 mbar) lors d'une émission complète du liquide à l'intérieur du réservoir de liquide (10) par la membrane (5).

5. Générateur d'aérosol selon l'une quelconque des revendications précédentes, dans lequel la pression négative initiale avant que la membrane (5) oscille est comprise entre 5 kPa et 35 kPa, de préférence 10 kPa et 20 kPa, et le plus préférentiellement entre 10 kPa et 15 kPa (50 mbar et 350 mbar, de préférence 100 mbar et 200 mbar et le plus préférentiellement entre 100 mbar et 150 mbar).

6. Générateur d'aérosol selon l'une quelconque des revendications précédentes, comprenant en outre un élément d'étanchéité (16) agencé sur une ouverture du réservoir de liquide (10) pour fermer de manière étanche le réservoir de liquide, le dispositif générateur de pression négative comprenant un élément coulissant (21) raccordé à l'élément d'étanchéité (16) de telle sorte qu'un déplacement de l'élément coulissant (21) a pour effet un déplacement d'au moins une section de l'élément d'étanchéité (16), de sorte que le volume initial V_{RI} du réservoir de liquide (10) est augmenté à V_{R} pour générer la pression négative initiale.

7. Générateur d'aérosol selon la revendication 6, comprenant en outre un élément rotatif (26) raccordé à l'élément coulissant (21) de telle sorte qu'une rotation de l'élément rotatif a pour effet un déplacement sensiblement linéaire de l'élément coulissant.

8. Générateur d'aérosol selon l'une quelconque des revendications 1 à 5, dans lequel le réservoir de liquide est formé par une ampoule à insérer dans un logement du générateur d'aérosol et ouvrir par perçage pour un raccordement au premier côté de la membrane.

9. Générateur d'aérosol selon l'une quelconque des revendications précédentes, dans lequel le volume V_{R} du réservoir de liquide (10) contient plus de 11,5 ml de gaz, de préférence plus de 14,5 ml de gaz, et le plus préférentiellement plus de 16,5 ml de gaz.

10. Générateur d'aérosol selon l'une quelconque des revendications précédentes, dans lequel le rapport du volume du réservoir de liquide (10) V_{R} sur le volume initial V_{L} de liquide dans le réservoir de liquide (10) est supérieur à 2,4, et le plus préférentiellement d'au moins 2,8.

11. Générateur d'aérosol selon l'une quelconque des revendications précédentes, dans lequel la viscosité du liquide contenu par le réservoir de liquide (10) est d'au moins 1,5 mPa x s.

12. Générateur d'aérosol selon l'une quelconque des revendications précédentes, dans lequel le réservoir de liquide (10) comprend une marque d'étalonnage indiquant le volume initial V_{L} du liquide.
